# EUROPEAN PATENT APPLICATION

(11) **EP 3 581 189 A1**
(43) Date of publication of application: **18.12.2019**
(21) Application number: 18177815.0
(22) Date of filing: 14.06.2018
(51) Int. Cl.: A61K 31/727, A61P 9/10, A61P 7/02

(54) **MEDIUM MOLECULAR WEIGHT HEPARIN FOR TREATMENT AND SECONDARY PREVENTION OF ISCHEMIC STROKE**

(71) Applicant: Fytagoras B.V., 2333 BE Leiden (NL)
(72) Inventor: Welzel, Dieter, 90419 Nürnberg (DE)
(74) Representative: dompatent von Kreisler Selting Werner - Partnerschaft von Patent- und Rechtsanwälten mbB

(57) **Abstract**

It is the thorough consistency between the pathophysiological pattern of ischemic stroke and the haemostaseological characteristics of LD Heparin 10,5 Kd that confer specificity to the pertinent patent claim.
In particular it is specific role of Tissue factor (TF) and its complex with Factor Vila respectively trigging off cerebral thrombogenesis that condition the rationale of LD Heparin in ischemic stroke. It is the TF/F Vila - complex that makes up the adequate substrate for LD Heparin which is rather resistant to the classical antithrombin-mediated influence but primarily responsive to Tissue Factor Pathway Inhibitor (TFPI). Its extraordinary release on LD Heparin in which LMWH (Enoxaparin) is exceeded threefold constitutes the rationale of striking out on the new indication of stroke.
The TFPI-dependent impact is safeguarded by the controlled curtailment of anti-factor Xa activity as permissive for the inhibition of TF/F VIIa, the ratios of activities between TFPI and anti-factor Xa as expressed in the AUC's of both heparins differing threefold either.

It is not only that disease-centered antithrombotic effect but also the way how the risk of bleeding is tackled in general in which LD Heparin contrasts with all others in its class: Owing to the "double truncation" of the molecular spectrum i.e. through the curtailment of its low and very long molecular species major sources of bleeding are excluded.
The inventory of auxiliary properties in particular the adequate pharmacokinetics, the trimmed peak blood levels, the lack of plasmatic accumulation in age-dependent decline of renal function and renal insufficiency, the availability of valid and sensitive monitoring (APTT), and last but not least the prompt and complete reversibility of the anticoagulatory effect as contrasted to LMWH substantiates the indication-specific profile.
On the whole the multipronged refinements of LD Heparin are aiming at a heightened benefit-risk-ratio ("therapeutic window") which takes specific shape in cerebral thrombosis.
The patent claim associated reads: LD Heparin 10,5 Kd (10-11Kd) for the treatment and secondary prevention of ischemic stroke.

## Description

Stroke, a highly prevalent condition, exacts a substantial social toll in the form of the "Dreaded D's" i.e. the leading cause of chronic disability, the second leading cause of dementia, and the fourth leading cause of death in the United States. (1) Two thirds of all strokes occur in developing countries and over 8% of all stroke-related deaths occur just there.

As to the P.R. of China, the country has made substantial advances in medical research and patient care in recent decades, but its expanding economy, changes in lifestyle, and the rapidly aging population have resulted in a growing burden of noncommunicable diseases, i.e. diabetes, cardiovascular disease, and ischemic stroke with precedence. (2)

Arterial thrombosis is the dominant trigger of cerebral infarction and it can occur in the extra-and intra-cranial arteries when the intima is roughened and plaque forms along the damaged vessel.

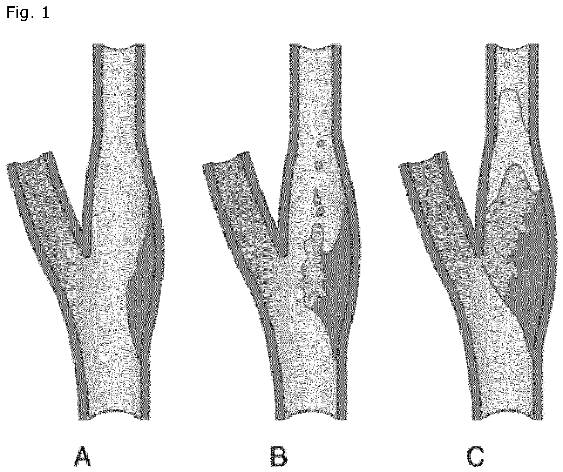

### Fig.1 (A) Plaque; (B) plaque with platelet emboli; (C) plaque with occlusive thrombus.

At present no proven treatment strategy exists except the intravenous application of recombinant tissue plasminogen activator (rtPA) as suggested by the National Institute of Neurological Disorders and Stroke (NINDS).

Thrombolytic drugs however can only be given to a defined patient population, and within a rather short time interval after onset of stroke symptoms.

The intention to use antithrombotic agents, particularly heparins, in the treatment of acute ischemic stroke is to inhibit thrombus propagation and embolization into more peripheral arterial segments. They facilitate thrombolysis and can prevent early occlusion after successful thrombolysis with rtPA.

The rationale to make use of heparins in acute stroke may look convincing, but clinical science so far does not support the routine application of antithrombotic agents.

It is because the heightened risk of bleeding associated undermines the endpoint benefit of anticoagulation.

Medium molecular weight heparin, herein after frequently called LD Heparin *10,5 Kd* exhibits the potential of shifting the therapeutic paradigm thus marking anticoagulation a proven strategy in ischemic stroke. That's the essence of the patent claim to be substantiated.

LD:" Low Dispersity" (see page 3)

Owing to "Cochrane Collaboration", reputated for its most comprehensive and best documented metanalyses in the major fields of medicine we are well informed on the state of the art:
The hypotheses of the Cochrane Stroke Group (3) were that immediate anticoagulation would be associated with:
1.) a reduced risk of dying or being dependent in activities of daily living a few months after stroke onset
2.) a reduced risk of early recurrent ischemic stroke
3.) an increased risk of symptomatic intracranial or extracranial haemorrhage, and
4.) a reduced risk of deep vein thrombosis and pulmonary embolism

Data from 21 trials including randomized data from >22000 participants showed that giving anticoagulants were not associated with *any* significant reduction in the odds of death at final follow up of greater than a month (OR 1.05; 95% Cl 0,98-1.12)

In respect to *recurrent stroke* during treatment, however, anticoagulation was associated with a statistically significant reduction in its incidence (OR 0,76; 95% Cl 0,65-0,88 an important result underscoring *basic* antithrombotic effectiveness of heparins.

Regarding symptomatic intracranial haemorrhage during treatment, immediate anticoagulation increased that type of complication (OR 2,55; 95% Cl 1,95-3,33). Regarding *both ie: any* recurrent stroke or symptomatic intracranial haemorrhage during treatment and during long-term follow up there were 11 trials including randomized data from >21000 participants.

"Treatment with anticoagulants reduced recurrent stroke, deep vein thrombosis and pulmonary embolism, but increased bleeding risk", so the authors' conclusion of the last Cochrane review in 2015. (3)

Although anticoagulation as compared to controls did not result in a *net reduction of the combined* incidence of stroke and intracranial haemorrhage, the (borderline) odds of this outcome (OR 0,97; 95% C3 0,85-1,1 implies the *rationale of refining anticoagulant therapy for stroke.*

So even some moderate improvement of tolerance would give the benefit-risk ratio a favourable turn. That's the perspective opened up to LD Heparin.

Anyhow the validity of the Cochrane analysis does not appear unconditional owing to the fact that *all* classes of anticoagulant were grouped together: unfractionated and low-molecular-weight heparin, (UFH/LMWH), heparinoids, thrombin-inhibitors and (even) oral anticoagulants which are regularly burdened with a significant bleeding problem. Against that conglomeration of dugs reflecting therapeutic arbitrariness LD Heparin 10,5 Kd stands out in embodying a pharmacological rationale that appears adjusted the patho-physiological peculiarities of ischemic stroke.

The next chapter presents the condensed characteristics of LD Heparin 10,5 Kd and its general impact regarding antithrombotic effectiveness and tolerance, preceded by an abridged, schematic representation.

### Haemostaseological characteristics of "Low Dispersity Heparin 10,5 Kd" (LD Heparin) as basis of the heightened benefit-risk ratio in ischemic stroke,

### (Schematic representation)

### Fig.2

### Low molecular heparin

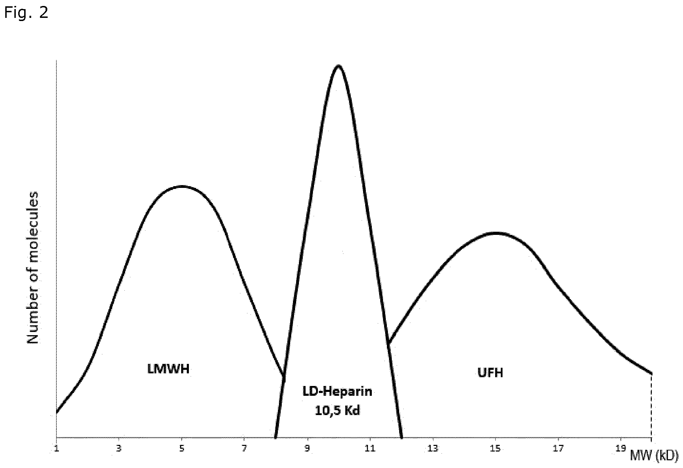

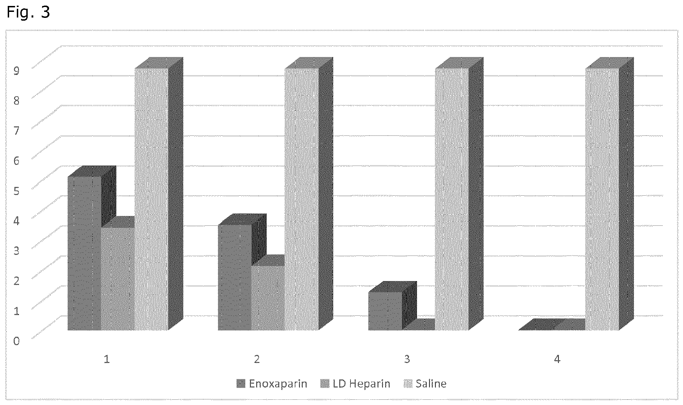

The anticoagulatory potential of LMWHs is curtailed by their low anti-thrombin activity (antFlla) and a rather modest TFPI-release in support of suppressing thrombin-formation. The pharmacodynamic deficiencies all the more carry weight since"...the most important pharmacological effect of *any* heparin is in fact inhibition of thrombin"(Z1) (Fig.2) A major problem area of LMWH refers to the abundance of so-called "low affinity" material (LAM) that is *principally* devoid of any anticoagulatory effect. (The percentage of LAM in enoxaparin amounts to 86%! ; Z2). Although LAM doesn't exhibit antithrombotic effectiveness it nevertheless induces bleeding: In a heparin-preparation including 85% LAM half of the heparin-related excess blood loss proved to be due to the latter (Z3). The modern insights into the mechanism of action of natural heparins focussing on the dominance of anti-thrombin-activity thus allowing to exclude LAM without loss in the anticoagulatory potential - are the clue to the rationale of LD-Heparin 10,5.

### Unfractionated heparin

The anti-thrombin activity of long chain heparin molecules (>11KD) is mainly restricted to the inhibition of fresh-generated fluid-phase thrombin. According to Ph. J. Hogg and Craig M. Jackson this molecular species promotes binding of thrombin to fibrin polymer thus curtailing its inactivation by the anticoagulatory heparin-antithrombin III-complex. This shortcoming explains the limited efficacy of long-chain heparin-species on thrombus-growth and the complications related i.e. extention of the ischemic zone ("stroke in progression") early recurrence, re-occlusion after treatment with fibrinolytic agents (Z4). Furthermore long-chain heparin-molecules exert a specific prohaemorrhagic impact not related to anti-thrombin activity as such (Z5). These facts suggested to exclude this material in LD-Heparin 10,5Kd.

Z1 M. Levi. Thrombosis and Haemostasis 2003; 1, 884.

Z2 A-V. Bendetowicz et al. Thromb & Haemostasis 1991; 67, 556.

Z3 Ockelford et al. Thrombosis Research 1982; 27, 679-690.

Z4 Ph. J. Hogg and C. M. Jackson J Biol Chemistry 1990; 265, 241.

Z5 D. Welzel, S. Beguin and H. C. Hemker Haemostasis 1999; 29, 170-178.

### The therapeutic rationale in the treatment of ischemic stroke

### Introductionary remarks

Therapeutic measures are "rational" if they are congruent to the pathophysiological mechanisms of the given disease.

Accordingly, it has to be demonstrated that LD-Heparin fulfils the requirements of *therapeutic specificity* in stroke.

Such specificity should show promise to overcome the insufficient antithrombotic response as peculiar to LMWH and UFH and to ensure the safety of anticoagulation.

Experimental efforts in giving the reasons for the disease-specific indication:
The primary task consists in elaborating LD-Heparin qualities that cover pathophysiological constellations being characterized by established thrombosis accompanied with the risk of extension.

Thereby emphasis his to be laid upon the spectrum of pharmacological and clinical-therapeutic impact factors directed at the critical dissociation between the anticoagulatory/antithrombotic influences on the one hand and the associated bleeding-potential on the other.

In the end special *auxiliary* properties of LD-Heparin are elaborated upon that support its peculiar positioning in ischemic stroke.

### LD Heparin 10,5 Kd: Basic pharmacological documentation (J. Fareed) The molecular definition, the antithrombotic, and the antihaemostatic Profile

Fareed J et al. Thromb and Haemost 1985; 11, 155-175.

The given heparin was prepared by controlled nitrous acid depolymerization followed by molecular filtration.

The HPLC - profile of the narrow-cut heparin and the in vitro-activities are given in Table 1 - with emphasis laid upon the anti-thrombin (anti-Ila) - inhibitory potential, and the reduced *molecular dispersity.*

**Table 1: HPLC Profile of LD Heparin 10,5 Kd**

| | | | |
|---|---|---|---|
| Number Average | 10,513 | | |
| Weight Average | 10,819 | | |
| Z Average | 11,233 | | |
| Peak Molecular Weight | 10,236 | | |
| Dispersity | 1,029 | | |

| In Vitro Activities of LD Heparin 10,5 as Compared to OFH and Enoxaparin | | | |
|---|---|---|---|
| | aXa U/mg* | alla U/mg | aXa / alia ratio |
| LD Heparin | 175 ① 1 | 170 | 1,03 |
| UFH | 160 ② 2 | 160 | 1,0 |
| Enoxaparin | 100 ① 1 | 26 | 3,85 |

| | | | |
|---|---|---|---|
| * anti-Factor Xa ① Measured by the 1^{st} international standard for LMWH ② Measured by the 4^{th} international standard for UFH | | | |

### Comparative antithrombotic activity of LD-Heparin and Enoxaparin in a "Rabbit Stasis Thrombosis Model"

The given model represents a modification of the original technique by S. Wessler which is based on the experimental combination of venous stasis in the jugular veins of dogs and the induction of a transient hypercoagulable, thrombophilic state in sequence of an infusion of heterologous canine serum.

The modification in question by *J. Fareed* an author of the given study includes white New Zealand Rabbits (instead of dogs) and FEIBA ("Factor Eight Inhibitor Bypassing Activity") as inductor of hypercoagulability. (4)

The description in the basic publication permits to leave it at some explanatory remarks: The jugular veins are surgically isolated with collateral vessels being cauterized. A 2-3cm segment of each jugular vein is isolated which includes the bifurcation of the vein. Silk ligatures get tied loosely around each tributary of the jugular veins without interference with the blood circulation.

FEIBA (7,5 U/kg) injected afterwards is allowed to circulate for 20 seconds. In rapid succession, the ligatures around both jugular veins are quickly tied, stasis being induced for exactly 10 minutes.

Then hemostats are placed outside the ligated area of each vein next to all ligatures. The vein segments are removed and opened lengthwise with scissors and visually checked for clot formation.

Clot grading is on a scale from 0 to + 4 (0, +1, +2, +3, +4), with a +4 meaning complete clot formation with no free erythrocytes. The resulting "Clot Score" describes the essential clot-formation.

In addition to the *plus symbol* assignment a numerical one is given to the clots ranging from 0 to 10 in order to allow computer analysis of clotting data.

The animals were subjected to treatment with either LD-Heparin 10,5 Kd or enoxaparin or saline respectively.

The administration of the drugs was by the intravenous route (5 U/kg, !0 U/kg, 25 U/kg, or 50 U/kg respectively) and by the subcutaneous one (25 U/kg, 50 U/kg, 75 U/kg, or 250 U/kg respectively).

The latency periods between administration of the test-drugs and the injection of FEIBA were 30 minutes after their intravenous application and two hours after the subcutaneous one.

### Results:

1) Antithrombotic effects of intravenously applied LD Heparin and Enoxaparin as manifested in the reduction of the Clot-Score.
The findings pertinent to both heparins and the control-series are demonstrated in the tables 2a-2c and in fig.2
The differences in the Clot-Scores favouring LD-Heparin are statistically significant with regard to 5U/kg (p ≤ 0,05) and 25U/kg (p ≤ 0,01)

**Table 2a) Saline control**

| | n = 10 |
|---|---|
| | 8,75 |
| | 7,50 |
| | 10,00 |
| | 10,00 |
| | 8,75 |
| | 7,50 |
| | 8,75 |
| | 7,50 |
| | 8,75 |
| | 10,00 |
| X ± S.D. | **8,70 ± 1,20** |

**Table 2b) Enoxaparin i.v.**

| | n = 10 | n = 10 | n = 10 | n = 10 |
|---|---|---|---|---|
| | 5 U/kg | 10 U/kg | 25 U/kg | 50 U/kg |
| | 5,30 | 2,50 | 2,50 | 0 |
| | 5,00 | 5,00 | 0 | 0 |
| | 4,90 | 3,75 | 1,25 | 0 |
| | 5,70 | 3,75 | 1,25 | 0 |
| | 6,00 | 2,50 | 1,25 | 0 |
| | 5,00 | 3,75 | 1,25 | 0 |
| | 5,10 | 2,50 | 2,50 | 0 |
| | 5,20 | 5,00 | 0 | 0 |
| | 4,80 | 3,75 | 0 | 0 |
| | 3,70 | 2,50 | 2,50 | 0 |
| X ± S.D. | 5,10 ± 0,58 | 3,50 ± 0,99 | 1,25 ± 1,02 | 0 ± 0 |

**Table 2c) LD Heparin i.v.**

| | n = 10 | n = 10 | n = 10 | n = 10 |
|---|---|---|---|---|
| | 5 U/kg | 10 U/kg | 25 U/kg | 50 U/kg |
| | 2,70 | 3,75 | 0 | 0 |
| | 3,80 | 1,25 | 0 | 0 |
| | 3,80 | 1,25 | 0 | 0 |
| | 4,00 | 2,50 | 0 | 0 |
| | 3,10 | 2,50 | 0 | 0 |
| | 2,90 | 1,25 | 0 | 0 |
| | 2,70 | 2,50 | 0 | 0 |
| | 2,60 | 2,50 | 0 | 0 |
| | 4,50 | 1,25 | 0 | 0 |
| | 4,00 | 2,50 | 0 | 0 |
| X ± S.D. | 3,41 ± 0,68 | 2,13 ± 0,84 | 0 ± 0 | 0 ± 0 |

**Comparative antithrombotic effects of LD-Heparin and Enoxaparin i.v.**

| | | | | |
|---|---|---|---|---|
| Enoxaparin | 5,1 | 3,5 | 1,25 | 0 |
| LD Heparin | 3,41 | 2,13 | 0 | 0 |
| Saline | 8,7 | 8,7 | 8,7 | 8,7 |
| Doses (U/kg) | 5 | 10 | 25 | 50 |

### Fig. 3

Reduction of the Clot Score means reduction of clot formation:
LD-Heparin exhibits significantly higher antithrombotic potency.

Antithrombotic effects of *subcutaneously* applied LD Heparin and Enoxaparin (Table 3a, 3b)

The differences between both heparins are significant with regard to the doses of 25 U/kg, 75 U/kg and 100 U/kg (p < 0,01 - 0,05).

**Table 3a) LD Heparin s.c.**

| **Clot Score** | | | | |
|---|---|---|---|---|
| n = 10 | n = 10 | n = 10 | n = 10 | n = 10 |
| 25 U/kg | 50 U/kg | 75 U/kg | 100 U/kg | 250 U/kg |
| 4,20 | 2,50 | 2,20 | 0 | 0 |
| 3,70 | 2,50 | 1,70 | 0 | 0 |
| 4,10 | 3,75 | 2,10 | 0 | 0 |
| 4,00 | 2,50 | 2,00 | 0 | 0 |
| 3,20 | 2,50 | 1,40 | 0 | 0 |
| 3,90 | 3,75 | 1,30 | 0 | 0 |
| 4,20 | 2,50 | 1,70 | 0 | 0 |
| 3,50 | 2,50 | 2,50 | 0 | 0 |
| 3,50 | 2,50 | 1,50 | 0 | 0 |
| 4,00 | 3,75 | 2,00 | 0 | 0 |
| 3,83 ± 0,34 | 2,90 ± 0,60 | 1,84 ± 0,38 | 0 ± 0 | 0 ± 0 |

As to the control-series see table 2a

**Table 3b) Enoxaparin s.c.**

| **Clot Score** | | | | |
|---|---|---|---|---|
| n = 10 | n = 10 | n = 10 | n = 10 | n = 10 |
| 25 U/kg | 50 U/kg | 75 U/kg | 100 U/kg | 250 U/kg |
| 5,00 | 2,50 | 3,10 | 3,50 | 0 |
| 5,80 | 5,00 | 2,70 | 1,25 | 0 |
| 5,30 | 3,75 | 3,60 | 2,50 | 0 |
| 5,60 | 3,75 | 2,80 | 2,50 | 0 |
| 5,90 | 3,75 | 2,10 | 2,50 | 0 |
| 4,90 | 3,75 | 2,90 | 2,50 | 0 |
| 5,30 | 2,50 | 3,00 | 2,50 | 0 |
| 5,40 | 5,00 | 2,50 | 1,25 | 0 |
| 5,50 | 3,75 | 3,75 | 2,50 | 0 |
| 5,30 | 3,75 | 2,50 | 3,75 | 0 |
| 5,40 ± 0,32 | 3,75 ± 0,83 | 2,90 ± 0,50 | 2,50 ± 0,83 | 0 ± 0 |

The saline-controls are based upon the i. v. -series (s. table 2)

### 2) The haemorrhagic potential of LD-Heparin and Enoxaparin-as assessed upon in two "Bleeding Models"

### a) "Rabbit Ear Bleeding Model"

That model can serve to quantify the haemorrhagic effect of anticoagulatory agents. It originates in the technique proposed and described in detail by *I. F. Cade* (5)

So it can be left with some explanatory and complementary remarks:
The rabbit's ear is immersed in an isotonic saline bath at 37°C.

In using transillumination an area is selected free of major blood vessels for placing 5 standard-sized incisions.

The amount of blood lost within 10 min. is measured by counting the total number of red blood cells (RBC) in the immersion fluid of a defined volume.

The rabbits were treated with 25U/kg, 50U/kg, 100U/kg, 250U/kg and 500U/kg LD-Heparin, Enoxaparin or a saline solution (control) respectively by intravenous injections 5 minutes before performing the measurements.

Bleeding effects (Table 4a-4c)

**Table 4a) Saline:**

| | RBCx10⁹/L |
|---|---|
| | 0,08 |
| | 0,09 |
| | 0,06 |
| | 0,10 |
| | 0,12 |
| | 0,09 |
| | 0,10 |
| | 0,10 |
| | 0,11 |
| | 0,10 |
| X ± S.D. | **0,10 ± 0,02** |

**Table 4b) LD Heparin:**

| | 25 U/kg | 50 U/kg | 100 U/kg | 250 U/kg | 500 U/kg |
|---|---|---|---|---|---|
| | RBCx10⁹/L | RBCx10⁹/L | RBCx10⁹/L | RBCx10⁹/L | RBCx10⁹/L |
| | 0,06 | 0,13 | 0,13 | 0,17 | 0,25 |
| | 0,10 | 0,10 | 0,14 | 0,19 | 0,22 |
| | 0,10 | 0,10 | 0,11 | 0,20 | 0,26 |
| | 0,13 | 0,15 | 0,15 | 0,18 | 0,23 |
| | 0,14 | 0,14 | 0,10 | 0,15 | 0,21 |
| | 0,14 | 0,16 | 0,11 | 0,10 | 0,24 |
| | 0,14 | 0,14 | 0,14 | 0,11 | 0,27 |
| | 0,10 | 0,15 | 0,16 | 0,12 | 0,29 |
| | 0,08 | 0,13 | 0,18 | 0,14 | 0,28 |
| | 0,12 | 0,15 | 0,18 | 0,18 | 0,25 |
| X ± S.D. | 0,11 ± 0,03 | 0,14 ± 0,02 | 0,15 ± 0,03 | 0,17 ± 0,4 | 0,25 ± 0,03 |

**Table 4c) Enoxaparin:**

| | 25 U/kg | 50 U/kg | 100 U/kg | 250 U/kg | 500 U/kg |
|---|---|---|---|---|---|
| | RBCx10⁹/L | RBCx10⁹/L | RBCx10⁹/L | RBCx10⁹/L | RBCx10⁹/L |
| | 0,13 | 0,14 | 0,10 | 0,28 | 0,44 |
| | 0,09 | 0,12 | 0,09 | 0,25 | 0,46 |
| | 0,10 | 0,12 | 0,14 | 0,26 | 0,36 |
| | 0,12 | 0,14 | 0,12 | 0,23 | 0,28 |
| | 0,08 | 0,10 | 0,12 | 0,22 | 0,28 |
| | 0,14 | 0,10 | 0,10 | 0,29 | 0,33 |
| | 0,13 | 0,12 | 0,14 | 0,21 | 0,38 |
| | 0,12 | 0,13 | 0,13 | 0,27 | 0,40 |
| | 0,11 | 0,14 | 0,15 | 0,25 | 0,42 |
| | 0,10 | 0,12 | 0,13 | 0,24 | 0,32 |
| X ± S.D. | 0,11 ± 0,02 | 0,12 ± 0,01 | 0,12 ± 0,02 | 0,25 ± 0,03 | 0,37 ± 0,06 |

The finding on blood loss due to both heparins don't allow a differentiation up to 100U/kg, whereas the higher doses (250 and 500/kg) imply significant differences

(p ≤ 0,001 - p ≤ 0,05) in favour of LD-Heparin.

### b) "Rabbit Ear Template Bleeding Model"

That model is used for studying the haemorrhagic effects of anticoagulant and antiplatelet agents.

The principle: By use of a Simplate blade (Organon Teknika Corporation, Durham, NC) moved into position on the dosal area of the rabbit's ear the interconnecting venule between the central artery and the marginal vein is transsected.

A filter paper is used to blot the blood flow every 30 seconds until the blood no longer stains the filter paper.
In this way the *"total bleeding time"* is assessed.

For the given study the rabbits were treated with 100U/kg, or 250U/kg, or 500U/kg of LD-Heparin and enoxaparin respectively. The control group received the same volume of an isotonic saline solution.

Comparative bleeding effects of LD Heparin and Enoxaparin

### Fig. 4

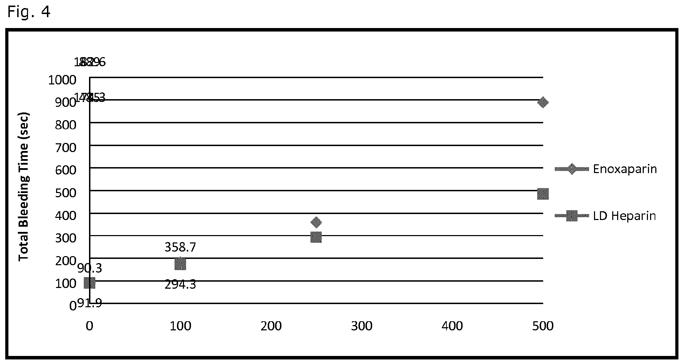

LD Heparin induces less bleeding than enoxaparin with the differences being statistically significant in regard to 250 U and 500 U / Kg. (Fig. 4, Table 5a-5c)

Bleeding effects: Total bleeding time (Table 5a-5c)

**Table 5a) Saline control**

| | |
|---|---|
| | 96 |
| | 86 |
| | 99 |
| | 60 |
| | 109 |
| | 92 |
| | 88 |
| | 96 |
| | 87 |
| | 90 |
| X ± S.D. | 90,30 ± 12,68 |

**Table 5b) LD Heparin**

| | 100 U/kg Seconds | 250 U/kg | 500 U/kg |
|---|---|---|---|
| | 156 | 366 | 388 |
| | 165 | 289 | 555 |
| | 230 | 234 | 434 |
| | 165 | 257 | 589 |
| | 182 | 367 | 423 |
| | 167 | 245 | 468 |
| | 178 | 256 | 488 |
| | 180 | 299 | 525 |
| | 158 | 309 | 456 |
| | 162 | 321 | 532 |
| X ± S.D. | 174,30 ± 21,57 | 294,30 ± 47,53 | 485,80 ± 63,69 |

**Table 5c) Enoxaparin**

| | 100 U/kg Seconds | 250 U/kg | 500 U/kg |
|---|---|---|---|
| | 170 | 399 | 867 |
| | 168 | 333 | 999 |
| | 243 | 367 | 1000 |
| | 169 | 360 | 828 |
| | 160 | 323 | 788 |
| | 188 | 344 | 823 |
| | 176 | 386 | 806 |
| | 165 | 348 | 924 |
| | 191 | 355 | 942 |
| | 196 | 372 | 920 |
| X ± S.D. | 182,60 ± 24,38 | 358,70 ± 23,32 | 889,70 ± 78,22 |

An additional control-series including 10 animals resulted in 91,9 ± 13,3 seconds (data not shown). LD-Heparin induces less bleeding than enoxaparin with the differences being statistically significant in regard to both the higher doses (p ≤ 0,01).

### 3) Discussion of the preclinical findings by J. Fareed

The detailed findings laid down in graphs and tables (Tables 1-5, Fig. 4) furnish consistent evidence of LD Heparin's superiority as against LMWH which pertains to antithrombotic efficacy and tolerance either.

The given experimental setting for the proof of antithrombotic activity corresponds to the pathophysiological pattern of *advancing "symptomatic" thrombosis* as pertinent to ischemic stroke.

As a matter of fact, Fareed's experiments fall in the range of *manifest* thrombogenesis reflected by *full-grown* thrombosis that go beyond the stage of mere hypercoagubility, in which the generation of thrombin and its decay are still in balance. Consequently, it can be considered a major advantage of LD in the indication that it fully matches symptomatic thrombosis.

To become symptomatic incipient thrombi are first subject to extension by accretion of fibrin, which bring up a special therapeutic problem: Fibrin restricts the anticoagulatory action of unfractionated heparin, the long-chain molecules of which are bridged to thrombin, thereby occupying critical binding sites necessary for its neutralisation by the AT III-heparin complex.

LD Heparin however, due to its sharp-cut molecular profile i.e. by the exclusion of long-chain molecules is not subject to this type limitation of its anticoagulant action: "Heparin species of molecular weight <11,200D are much less effective in promoting thrombin-binding to fibrin" than the high-molecular weight ones, see J. Hogg. (Z. 4, Fig. 2)

Against this background the question may arise *why* established LMWH can't substitute for UFH in this respect:
It is because of their molecular-weight-dependent, genuinely much weaker antithrombin activity,
insufficient in order to counteract advancing thrombosis optimally.
Thus the specified advantage of LMWHs shorter heparin chains cannot compensate for the relative deficiency in antithrombin activity.

*LD Heparin, lends itself for overcoming the therapeutic dilemma regarding "symptomatic" thrombosis. On the one hand its molecular profile doesn't favour thrombin-binding to fibrin on the other its anti-thrombin-activity is an unparalleled one.*

That peculiarity of LD Heparin would gain special importance in the framework of "thrombolytic therapy in order to prevent recurrent thrombosis dues to the (fully retained) coagulatory activity of fibrin-bound thrombin.

*In essence the results give verifiable reasons for the heightened benefit-risk ratio of the product as against LMWH which paves the way for the indication "ischemic stroke".*

In addition of primary importance in ischemic stroke is the *detachment* of LD Heparin's antithrombotic action from its influence upon bleeding i.e. the differential impact upon the outcome measures as compared to LMWH is of fundamental significance.

*Such type of difference between various type heparin, was never demonstrated before.*

*The conclusion is downright: In respect to the weaker anticoagulatory*/*antithrombotic efficacy of enoxaparin the enhanced bleeding-intensity associated can only be due to non-anticoagulatory, nevertheless bleeding-inducing molecules (see below).*

LD Heparin's superior tolerance is based on the *exclusion* of such type harmful molecules with the effect that *bleeding is curtailed despite of increased anticoagulatory*/*antithrombotic efficacy.*

*Such improved benefit*/*risk ratio permits differentiated dosage-regimens according to whether the risk of advancing thrombosis or bleeding is considered the dominant one. Such flexibility in the therapeutic approach as key to the treatment for stroke need be elaborated upon explicitly.*

For that purpose, the antithrombotic efficacy of LD Heparin and enoxaparin were defined in terms of the *equi-tolerable doses,* and the bleeding effects in terms of the *equi-effective (antithrombotic)* ones.

Correspondingly, regression curves according to Fareed's findings as related to the "clot-score", "bleeding time", and "blood loss" were established leading to :
1.) ***Equi-effective*** doses according to the "clot-score model":
   LD Heparin dose = 0,56 enoxaparin dose
2.) ***Equi-tolerable*** doses according to the "blood-loss model":
   LD Heparin dose = 1,78 enoxaparin dose,
   and according to the "bleeding time model":
   LD Heparin dose = 1,58 enoxaparin dose

*In accordance with this analysis the therapeutic dose of LD Heparin could be increased by 58-78% or decreased by 44% in comparison with enoxaparin without cuts in tolerance or efficacy respectively as against that benchmark LMWH.*

Thus, LD Heparin is equipped with a unique *"pharmacodynamic reserve"* utilizable for the benefit of special target-populations e.g. for patients with an excessive risk of bleeding.

Such adaptability in measuring out individual dosages substantially expands the therapeutic usefulness in the indication concerned, which is an adjunct argument for special claim in ischemic stroke.

The experimental findings included the dose-regimen envisaged for the treatment in ischemic stroke (150-200 mg/kg daily) which is identical to what is used in treating deep vein thrombosis and the acute coronary syndrome.

That comparability of the dosage-regimen in the preclinical documentation with that of the established clinical-therapeutic one doubtlessly benefits the relevance of the results.

Even if there were dose-related differences in the anticoagulatory response between the experimental species chosen and men-up to the proof of the contrary-they would hardly interfere with the differential outcome on both heparins.

### Human-pharmacological correlates to the animal-experimental ones in regard to basic principles of anticoagulation in ischemic stroke

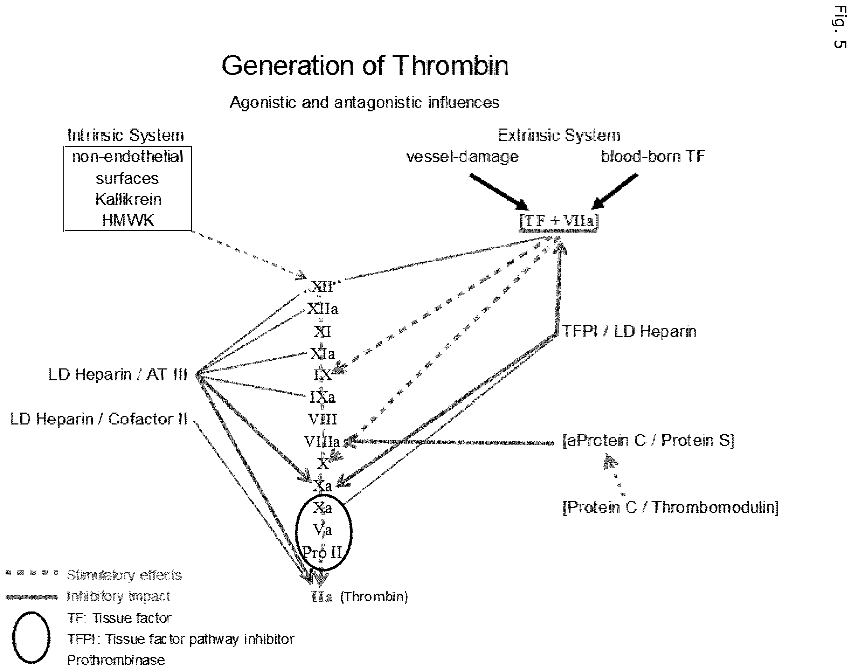

Heparins of natural origin, act first and fore-most via their antithrombin - (antFlla) effect. (Fig.5)

That's the state of the art in heparinology based upon the pioneering research of H C Hemker, Maastricht, and his school.

As to LD-Heparin, it exhibits "the highest in-vivo antithrombin activity on a molar and per-mg basis and there are good reasons to believe that LD-Heparin is the *"pure specifically working core of any natural heparin preparation," a statement of H C Hemker himself. (6)*

"It may therefore be more efficient and possibly also safer than other heparins, especially because substantially lower doses (on a weight and a molar basis) are sufficient to bring about affects comparable to those of UFH and LMWH."

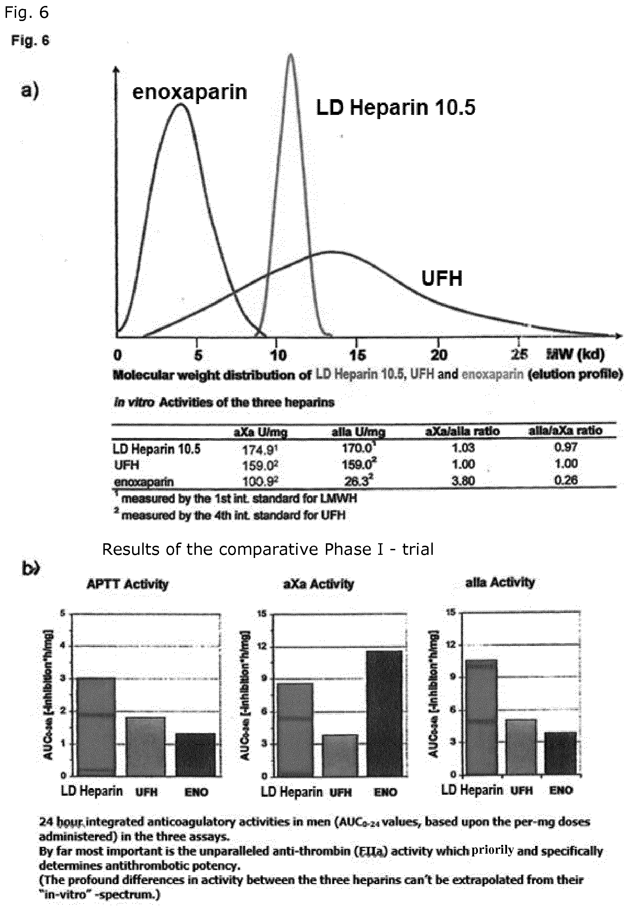

As a matter of fact, the 24 hour integrated antithrombin (aIla) effect of LD-Heparin in men amounts to 260% and 200% as compared to LMWH (enoxaparin) and UFH respectively. (Table 6, Fig.6)

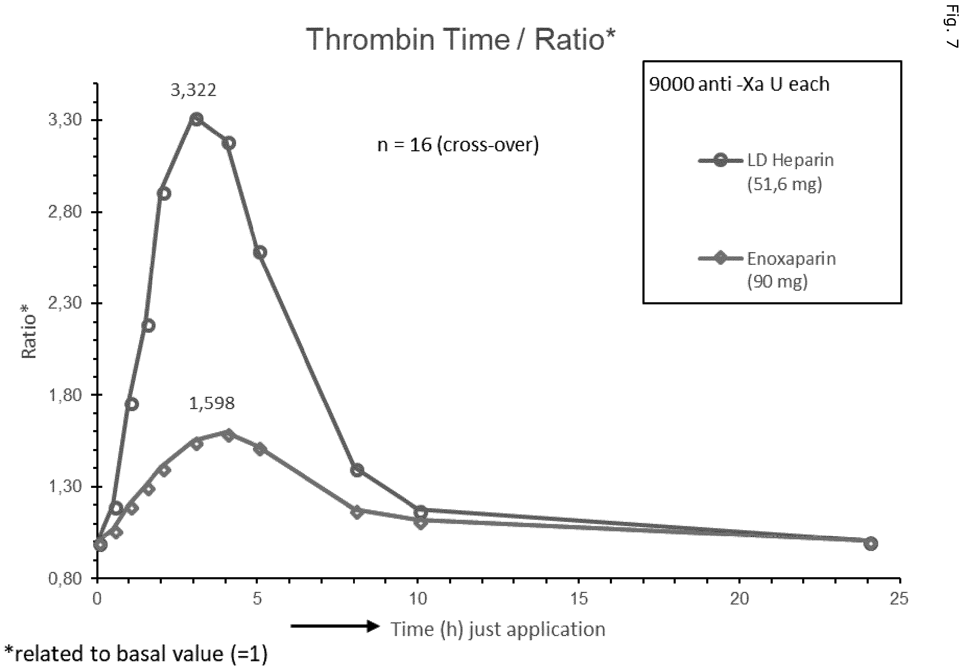

The "Thrombin Time" (TT) a specific anti-Ila-test, evidences the unique superiority of LD-Heparin alike (Fig.7), the same holding true for the "Activated Partial Thromboplastin Time" (APTT), which measures to what extent *endogenous thrombin* is inhibited by heparins. (Table 6)

The differential activity-profiles and the per mg-doses associated signalize that obviously substantial molecular species can be excluded devoid of anticoagulatory activity but nevertheless inducing bleeding (see below).

### Fig. 5

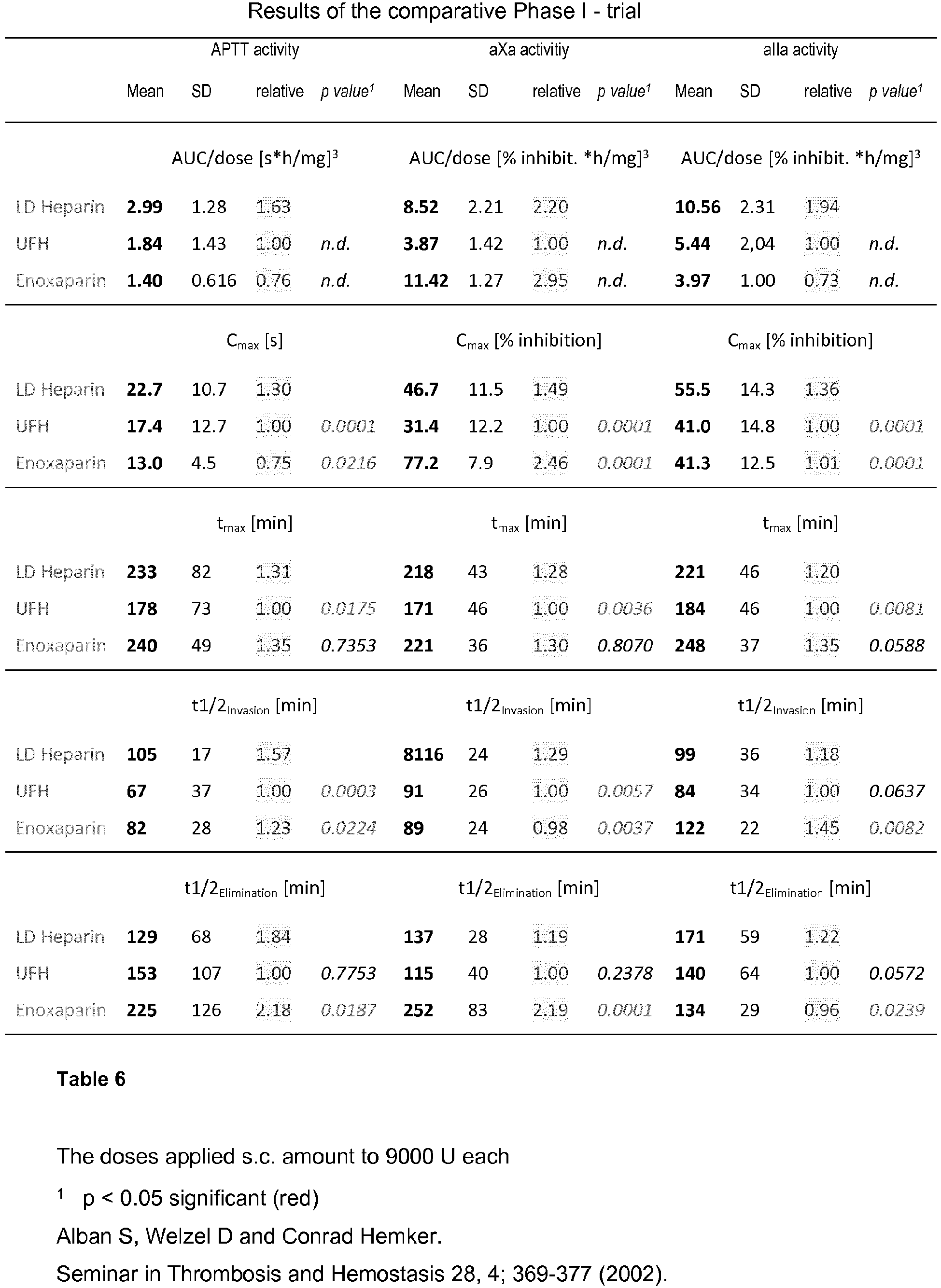

### Fig. 6

### Fig. 7

### Human pharmacological correlates ..... continued

There is even *more* to infer from that clinical-pharmacology study: If one realizes that bleeding due to heparin is a function of the *actual blood-level,* and the *peak* one especially it becomes clear that LD Heparin represents the only heparin-species the pharmacokinetics of which resembles that of a *sustained release preparation*:
From table 6 it emerges that LD Heparin and enoxaparin in terms of anti-Ila activity differ in respect to the given ratio between the AUC (0-24h) dose and the peak activity (max % inhibition).
So patients could be exposed to an increased antithrombotic dose-regimen as ceteris paribus linked together with a *pharmacokinetical-based* reduced bleeding risk.

### Tissue factor (TF) and "Tissue Factor Pathway Inhibitor" (TFPI), in ischemic stroke, getting to the core of the therapeutic specificity of LD Heparin

### I. Tissue factor (TF)

It is well established that the prime trigger to blood coagulation in vivo is "Tissue factor" that is exposed on the surface of fibroblasts and pericytes in the adventitia and media smooth-muscle cells of the vessel wall. The endothelium acts as a barrier separating TF from the flowing blood thus preventing the initiation of blood-clotting in the absence of injury or vessel-damage.

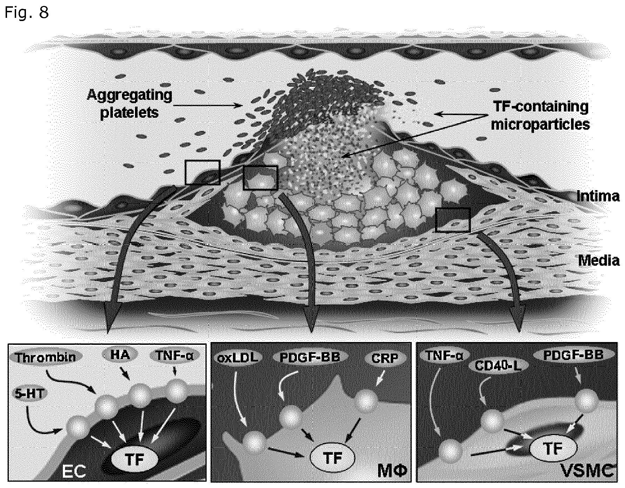

TF is also present as a "blood-born species" associated with "microparticles" in the circulating blood. These particles are derived from leucocytes and monocytes. During thrombus formation platelets accumulate at the vessel wall and express P-selectin, an adhesion molecule that binds to microparticles allowing the thrombus to capture microparticles carrying TF. Fibrin propagation i.e. thrombus growth is *dominated* by blood-born TF. (Fig.8)

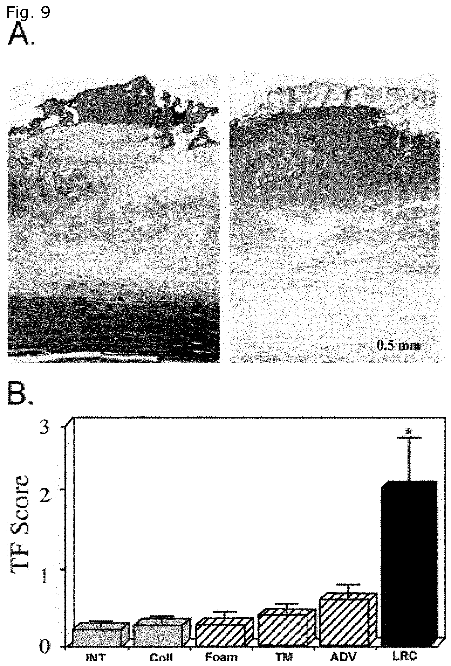

TF has been identified in human atherosclerotic coronary and carotid plaques by immunohisto-chemistry in several cell types, such as monocytes, foam cells, and fibroblasts. Furthermore, it has been shown that TF present in human atherosclerotic plaques retain its full procoagulant properties. (Fig.9)
Taken together there is abundant evidence underlining the importance of TF and the TF/FVIIa complex as the *main determinant of human atherosclerotic plaques* thrombogenicity.
"The inhibition of TF pathway seems to be one of the most promising interventions to stop the thrombotic process." (P. Golino) (Lit. page 22).

### Fig. 8

| |
|---|
| Tissue factor in the atherosclerotic plaque. In the environment of atherosclerotic plaques, (TF) is present at high levels in endothelial cells, vascular smooth muscle cells, macrophages/foam cells, and in the necrotic core. TF induction is exemplified by selected mediators in endothelial cells (EC, left panel), macrophages (Mϕ, middle panel), and vascular smooth muscle cells (VSMC, right panel). |
| On plaque rupture, highly procoagulant material including TF-containing microparticles is released into the blood, leading to rapid initiation of coagulation, platelet aggregation, and, ultimately, thrombus formation with vessel occlusion. |
| J. Steffel, Thomas F. Lüscher and Felix C. Tanner, |
| Tissue factor in Cardiovascular Diseases ..... |
| Circulation 2006; 113, 722 - 731. |

### Fig. 9

| |
|---|
| Panel A: Representative low-power photomicrographs of human atherosclerotic plaques exposed to flowing blood. Left photomicrograph shows connective tissue stain of a plaque with lipid-rich atherosclerotic lesion. Right photomicrograph shows an adjacent section of the same specimen specifical stained for TF. The brown color represents positivity of the reaction and, therefore, indicates presence of TF. It can be appreciated that most the intense staining is present in the lipid-rich core. Panel B: TF activity score. The atherosclerotic plaque was positioned in a perfusion chamber and the procoagulant activity of TF was evaluated by measuring the amount of FXa generated. TF staining intensity by immunohistochemistry was directly correlated to the procoagulant activity of the plaque, indicating that TF great contributes to the thrombogenicity of the atherosclerotic plaque. INT indicates normal intima; Coll, collagen-rich matrix; Foam, foam cell-rich matrix; TM normal tunica media; ADV, adventitia; and LRC, lipid-rich core. *p=.0002 |
| Golino P. Thromb Res. 106(3): V 25-65 (2002) |

### II.) Tissue Factor Pathway Inhibitor (TFPI): its influence upon blood-coagulation, and the effects of LD Heparin

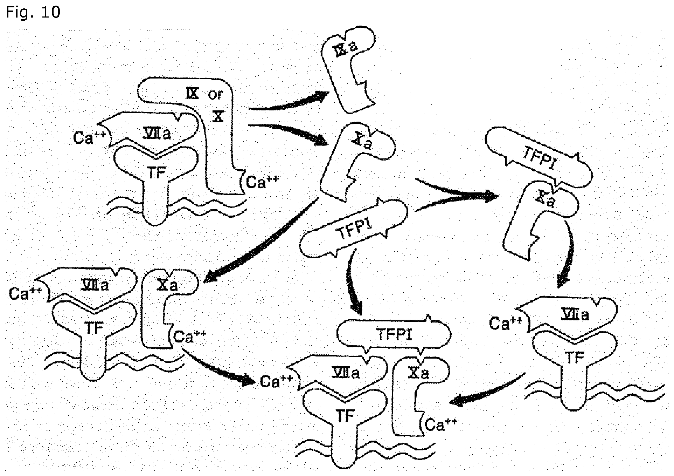

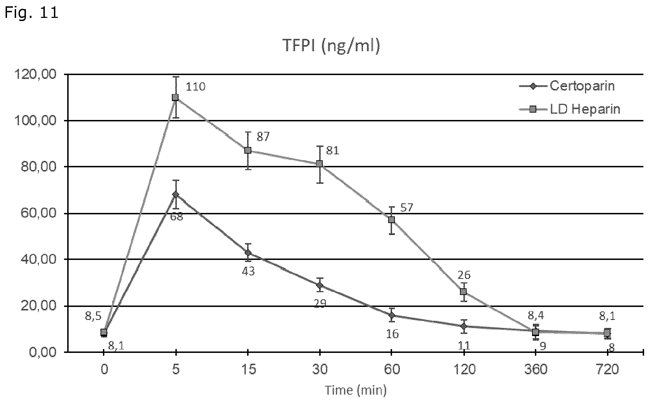

Pharmacology of TFPI
TFPI represent an endogenous anticoagulant protein that inhibits the initial phases of the proagulant response Fig. 5
Circulating plasma TFPI is partly bound to lipoproteins as carrier. Only the carrier-free inhibitor ("free TFPI"), however, has a major influence on blood-coagulation. TFPI inhibits both FXa and TF/F Vila in forming a stable quarternary complex. (Fig. 5,10)
"TFPI" contributes about one third to the overall anticoagulatory potential of heparin". (7)
According to a recent publication *the neutralization of* free TFPI in normal human plasma (22 ng/ml) by an anti-TFPI antibody resulted in a 25% increase in thrombin generation. (8)
As expected the neutralization of higher TFPI-levels result in a (considerably) stronger impact on thrombin-generation. (8)
So even modulation of the physiological TFPI-concentration translates into a substantial haemostaseological impact.
These finding, impressively underline the suppressive function of TFPI in controlling hypercoagubility and thrombogenesis associated.

The anticoagulatory-impact of TFPI is a highly distinct one all the more because the blockade of the TF/FVIIa function in contrast to direct thrombin inhibition results into some substantial thrombo-prophylactic action *without* compromising hemostasis i.e. inducing bleeding (see added abstract).

### Fig. 10

| Thromb Haemost. 1997 Sep;78(3): 1142-9. |
|---|
| **Dissociation of antithrombotic effect and bleeding time prolongation in rabbits by inhibiting tissue factor function.** |
| Himber J¹, Kirchhofer D, Riederer M, et al. |
| Abstract |
| Inhibition of the tissue factor/factor VIIa (TF/F.VIIa) complex attenuates thrombosis in different animal models of arterial thrombosis. However, it remains unclear to what extent the antithrombotic effects are associated with changes in hemostatic functions and how this compares with inhibition of thrombin, an enzyme acting at a later stage in the coagulation cascade. The antithrombotic and the antihemostatic effects of a monoclonal anti-TF antibody (AP-1) were compared in a model of arterial thrombosis to those of a direct thrombin inhibitor (napsagatran) and heparin. In anesthetized rabbits transient arterial thrombi were induced by mechanical damage to the subendothelium of a moderately stenosed carotid artery. Recurrent formation and dislodgement of thrombi resulted in cyclic flow variations (CFVs) which were monitored over 2 hours. Rabbits received intravenously either a placebo (control), a monoclonal anti-rabbit TF antibody (AP-1, 0.05 mg/kg as an i.v. bolus repeated every 15 min, a specific low molecular weight thrombin inhibitor (napsaqatran, 3 microg/kg/min) or heparin (3 and 13 microg/kg/min). The effect of the inhibitors on the hemostatic system was studied in a separate set of rabbits by measuring template bleeding times (BT) in the ear arterioles, marginal ear vein and the nail cuticle of the foreleg. AP-1 and napsagatran showed a similar antithrombotic activity (78% and 80% abolition of the CFVs, respectively), whereas either low or high dose heparin was poorly effective (43% and 40% inhibition of CFVs, respectively). At these antithrombotic doses and even at 4-fold higher dosage, AP-1 did not significantly alter the BT, whereas napsagatran and heparin prolonged the ear vessels and cuticle BT in a dose-dependent manner. These results suggest that in contrast to direct thrombin inhibition, the blockade of the TF/F. VIIa function did not result in a concomitant prolongation of the bleeding time. Thus, dissociation of antithrombotic and antihemostatic effects indicates that inhibition of the coaqulation system at its initial stage represents a promising approach for the development of new anticoagulants. |

LD Heparin distinguishes from all other heparins, LMWH and UFH alike, by the *extra-ordinary* release of free TFPI in the circulation. (see below) which w'll be demonstrated in the following.

First, comparative animal-experiments on LD Heparin and the LMWH certoparin (MW 4200-6200D) are put forward the gravimetrical based dose regimen being 1 mg/Kg (J Fareed 2015).

The daily therapeutic dose of certoparin in men comes to 16000 U

### Fig. 11

The emerging superiority of LD Heparin appears exceptional.

What really counts however, is the comparison with enoxaparin as the benchmark LMWH, which is indicated all the more because all anticoagulatory agents developed during the last 25 years were exclusive by based upon that type of comparison, (hirudin, rivaroxaban, dabigatran, apixaban etc.
So the overall judgement on LD Heparin ultimately depends on its differentiation from enoxaparin in men preferably.

### TFPI-release in men

Prominence is given to a randomized, double-blind, multiple cross-over trial on 16 healthy male subjects, carried out by Prof. Dr. S. Alban, (L) Kiel Accordingly, LD Heparin brings about a *3fold* higher plasma-concentration of "free" TFPI as compared to enoxaparin (Fig.9)

Dose-standardized AUC 0-24h values on the basis of the concentrations of TFPI released

In order to illustrate the weight-specific potency of the heparins to mobilize TFPI the dose-standardized AUC (0-24h) were calculated.

The AUC both for total and for free TFPI are *by far* the highest for LD Heparin. The ability of this relatively long-chain heparin to mobilize TFPI is significantly more pronounced than that of short-chain enoxaparin. Morever, due to its better bioavailability, it also exceeds the action of UFH.

S. Alban, Kiel Phase I - trial

### Fig.12

AUC₀₋₂₄ₕ values calculated from the release of total and free TFPI antigen (concentration minus the baseline level) and weight-specific AUC₀₋₂₄ₕ values (AUC₀₋₂₄ₕ divided by the mg dose administered) of LD Heparin, (UFH) and enoxaparin (ENO) in the three assays.

*It is the anticoagulatory function of TFPI and the related impact of LD Heparin respectively that constitute a novel entity of heparin lending itself for the secondary prevention and treatment of ischemic stroke which w'll be elaborated below.*

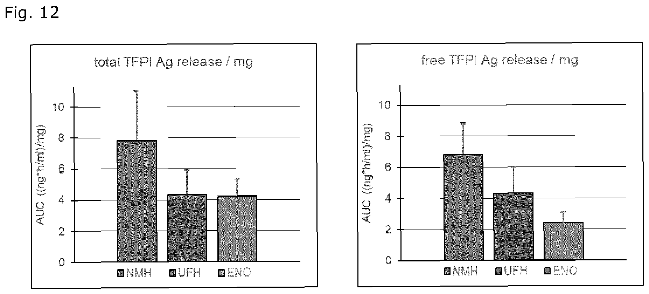

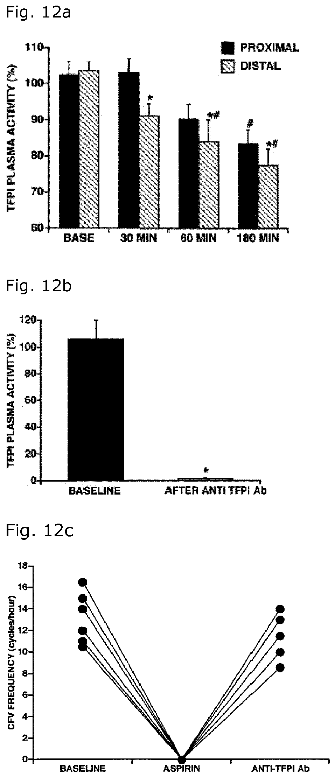

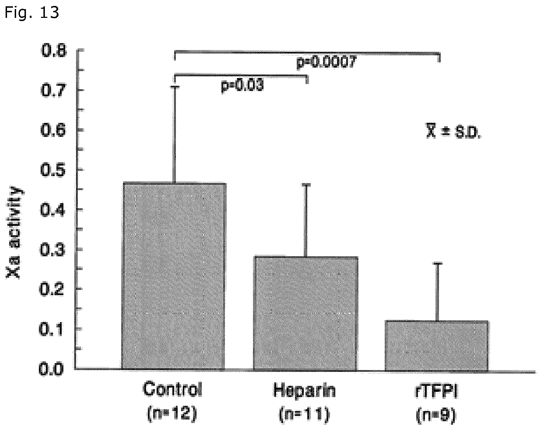

That novelty isn't exhausted in the enhanced release of "free" TFPI:
A *unique* dimension of distinctiveness originates in the *differential* ratio of TFPI - and anti-factor-Xa activities on both heparins AUC (ng x h/ml)/mg):
AUC [% inhib. x h/mg, see Table 6, Fig. 12] in which LD Heparin exceeds LMWH more than 3 times.

What pharmacological importance is to attach to that striking difference? :
LMWH due to its excessive anti-factor Xa - activity can trap factor Xa - molecules thus preventing the formation of the TFPI factor Xa - complexes as necessary for the inhibition of factor Vila / TF. (see Fig. 5 and 10)
"This mechanism could help explain the relative inefficiency of heparin in such disease states in which tissue factor is thought to play an important role, that's the statement of P. M. Morton, the pioneer in research on TFPI. (9)

### Animal-experimental and pathophysiology-related evidence of TFPI's function in atherosclerotic disease

Atherosclerotic plaque rupture is the main trigger mechanism in ischemic stroke, and TFPI represents a general principle in suppressing or curtailing the resulting thrombosis.

The following publications furnish unequivocal evidence of endogenous TFPI participating in pathogenesis of atherosclerotic thrombosis and how its dynamics is controlled and curtailed by TFPI in *physiological* concentration.

The lucidity of two abstracts, (**I**, **II**) attached do best justice to the scientific findings.

**I.**

| Endogenous Tissue Factor Pathway Inhibitor Modulates Thrombus Formation in an In Vivo Model of Rabbit Carotid Artery Stenosis and Endothelial Injury |
|---|
| Massimo Ragni, MD; Paolo Golino, MD, PhD; Plinio Cirillo. |
| *Methods and Results:* Intravascular thrombosis was initiated by placing an external constrictor around endothelially injured rabbit carotid arteries (n=10). Carotid blood flow velocity was measured by a Doppler flow probe. After placement of the constrictor, cyclic flow reductions (CFRs), due to *recurrent thrombosis,* developed at the site of stenosis. Transstenotic TFPI plasma activity was measured in blood samples before induction of CFRs and after 30, 60, and 180 minutes of CFRs. TFPI plasma activity distal to the site of thrombosis was significantly lower than the corresponding proximal values at 30, 60, and 180 minutes of CFRs. In addition, a progressive decrease in TFPI plasma activity was observed in both the proximal and the distal samples, indicating consumption of TFPI during thrombus formation. In 10 additional rabbits, CFRs were abolished by administration of aspirin (10 mg/kg). In the animals in which aspirin abolished CFRs, endogenous TFPI was depleted by a bolus of a polyclonal antibody against rabbit TFPI, and the effects on restoration of CFRs were monitored. In 5 of 6 animals in which aspirin abolished CFRs, depletion of endogenous TFPI activity caused full restoration of CFRs. |
| ***Conclusions-***The data of the present study support the involvement of endogenous TFPI in the process of thrombus formation in vivo and its active role in modulating arterial thrombosis. (Circulation. 2000; 102,113-117.) |

The publication by M. Ragni, P Golino and Plinio Cirillo (abstract above) conveys that TFPI in passing the site of thrombus formation - is "utilized" in the process of neutralization of active clotting factor (TF/VIIa; FXa) which is reflected by the arterio-venous difference of its blood concentration. (Fig. 12a)

Moreover, it is shown that the inhibition of TFPI by specific antibodies (Fig. 12b) results in a *prompt* reactivation of (ongoing) thrombogenesis as initially suppressed by Aspirin: a direct proof of TFPI's inhibitory impact. (Fig. 12c)

| |
|---|
| **Fig. 12a** |
| Transstenotic TFPI plasma activity during *intracarotid* thrombus formation. TFPI activity was significantly lower in samples obtained from carotid artery distal to site of thrombus formation. Furthermore, TFPI activity progressively decreased over time in both proximal and distal samples, indicating consumption of TFPI at site of thrombus formation. **P*<0.05 vs corresponding proximal value; #P<0.05 vs corresponding baseline value. |
| **Fig. 12b** |
| Effects of anti-rabbit TFPI polyclonal antibody (Ab) on plasma TFPI activity. After administration of antibody, a marked decrease in plasma TFPI was observed. **P*<0.01. |
| **Fig. 12c** |
| Effects of depleting endogenous TFPI activity on spontaneous restoration of CFRs indicating recurrent thrombosis. In 6 rabbits, CFRs were inhibited by administration of aspirin (10 mg/kg) as an antithrombotic agent in arterial thrombosis. Thirty minutes after the CFRs were abolished, a goat polyclonal anti-rabbit TFPI antibody (Ab) was injected. Within 30 minutes, CFRs spontaneously restored in 5 of 6 animals. CFV indicates cyclic flow variation due to ongoing thrombogenesis. |

**II.**

| |
|---|
| Tissue Factor Pathway Inhibitor Attenuates Procoagulant Activity and Upregulation of Tissue Factor at the Site of Balloon-Induced Arterial Injury in Pigs |
| James St. Pierre et al. |
| Intravenous infusion of recombinant tissue factor pathway inhibitor (rTFPI) for 24 hours decreases neointimal thickening and luminal stenosis 1 month after balloon-induced injury to the *carotid arteries* in minipigs. This study was designed to determine whether the effect of rTFPI is accounted for by early decreases in procoagulant activity and thrombosis on the injured vessel wall. Vascular injury was induced by balloon hyperinflations in both carotid arteries of anesthetized pigs given no anticoagulant as a control, an intravenous infusion for 24 hours of rTFPI (0.5 mg/kg bolus and 25 *µ*g · kg-¹ · min⁻¹, n = 14), or an intravenous infusion of unfractionated heparin (100 U · kg⁻¹ · h⁻¹, n = 19). |
| Accumulation of radiolabeled autologous platelets was markedly decreased over 24 hours on injured arteries from animals given rTFPI (0.6x10⁶/cm²) compared with controls (2.5x1 0⁶/cm², *P* = 0.0004). "Factor Xa activity, was decreased more markedly on arteries from rTFPI-treated animals (0.14 ± 0.13) than those from heparin-treated animals (0.29 ± 0.18) compared with controls (0.47 ± 0.24; OD, *p* = 0.0007). |
| In addition, arteries from rTFPI-treated animals showed a 4-fold lower induction of tissue factor protein compared with controls (*P* = 0.0002)." (Fig. 13) |
| **Fig. 13** |
| Factor Xa on the luminal surface 24 hours after balloon hyperinflation-induced injury of the carotid arteries. Factor Xa expressed was decreased on vessels from both rTFPI- and heparin-treated animals compared with controls, although the decrease was more profound in rTFPI-treated animals. |
| (Arterioscler Thromb Vase Biol. 1999;19:2263-2268.) |

Complementary to these results it was shown in myocardial infarction in men that TFPI - in parallel to being utilized and "consumed" doing the passage of the coronary system neutralizes activated clotting factors as reflected by the increase of the TFPI/FXa complex in the coronary outflow.
*The rationale of heightening that antithrombotic impact by stimulating the release of free TFPI can hardly be better established.*

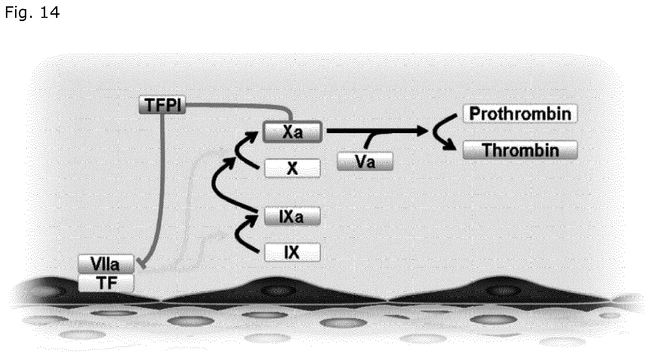

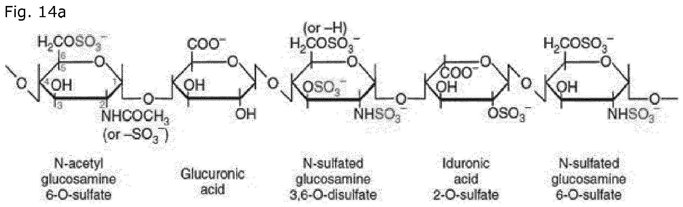

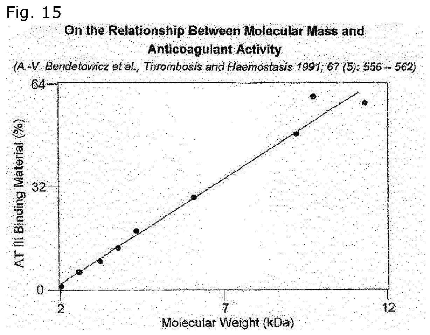

To sum up:
The thrombogenetic role of Tissue factor (TF) in atherosclerotic disease is indisputable. The same holds true for TFPI as an endogenous anticoagulant that controls thrombogenesis through suppressing thrombin generation a process in which TFPI is "consumed" as proven in men.
TFPI essentially attenuates the activity of the TF/FVII aV - complex as the trigger of coagulation, the scheme of J. Steffel and Th. Lüscher, both University of Zürich, giving a representative overview (Fig.14).
LD Heparin hold an eminent place regarding the release of "free" i.e. functionally active TFPI: In terms of the AUC 0-24h (ng / ml x h / mg) it *threefold* exceeds enoxaparin the concentrations of the antigen reaching blood levels of around 70ng / ml thus surmounting physiological levels threefold either.
That exceptional anticoagulatory impact acquires therapeutic specificity through its indifference toward normal haemostasis.

*It is this unique gap between LD Heparin's antithrombotic effectiveness on the one side and the mitigated bleeding risk on the other which will make LD Heparin the anticoagulant of choice in ischemic stroke, as a matter of fact for the first time opening up some systematic therapeutic access to this highly critical indication.*

Jan Steffel, Thomas F Lüscher et al.

### Tissue Factor in Cardiovascular Diseases

### Figure 14.

Tissue factor (TF) is a key initiator of the coagulation cascade. Formation of a complex with factor VIIa (FVIIa) leads to activation of factor IX (FIX) and factor X (FX), resulting in thrombin generation and, ultimately, clot formation. Tissue factor pathway inhibitor (TFPI), the endogenous inhibitor of TF activity, is synthesized and secreted mainly by endothelial cells. TFPI binds to FXa and thereby inhibits TF / FVIIa activity.

Circulation 2006;113,722-731.

### Beyond TFPI ...

### The multifactorial structure of heparin-related bleeding-complications.

What are the implication of LD Heparin's narrow-cut molecular profile?

To develop antithrombotics without compromising hemostasis is the guiding developmental principle.

Faced with the fact that the various heparins available so far don't differ in regard to their benefit-risk-ratio ("therapeutic window"), plausible reasons for the dissociation between LD Heparin's effectiveness and the product-related tendency towards bleeding should be furnished:

If antithrombotic activity and tolerance were *just* inversely and *invariably* associated with each-other then it were hardly conceivable that the former could be intensified without making allowances to the latter.

Rather surprisingly however, it was found out, that "bleeding" is only *marginally* correlated with anticoagulatory / antithrombotic effectiveness as (validly) expressed through the anti-thrombin (anti-Flla) - activity which necessarily draws the attention to "non-anticoagulant" / non-antithrombotic sources of bleeding. (Table 7)

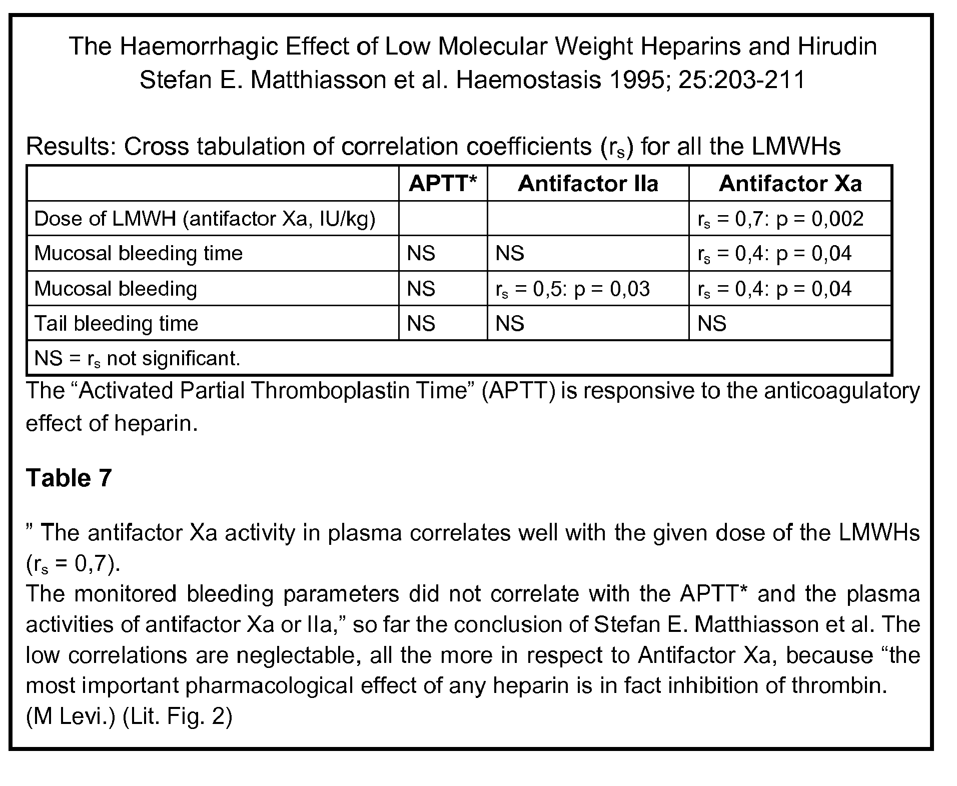

What are the sources of bleeding?

These sources can be *positively* identified, and in case of LD Heparin have been developmentally taken into account - with the emphasis upon a significant curtailment of "Low Affinity Material" (LAM) within the molecular spectrum of heparin (see the box)

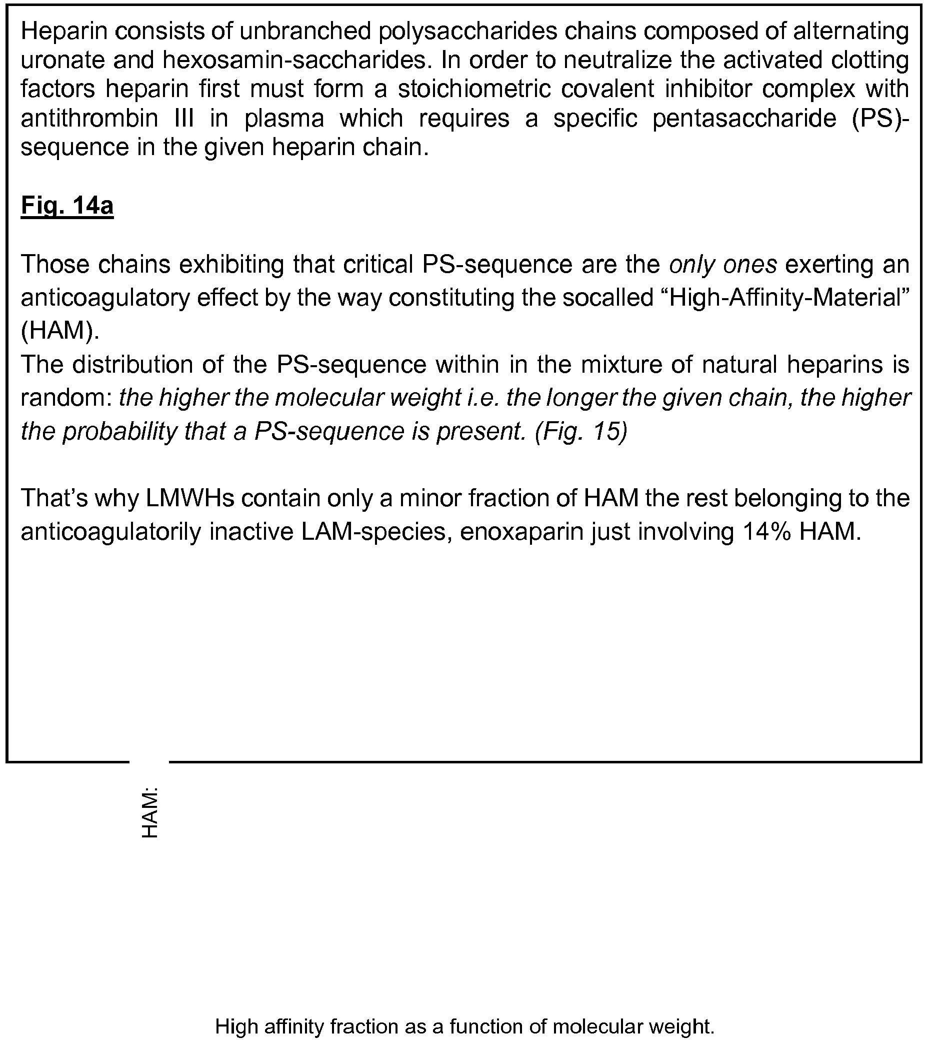

### Fig. 15

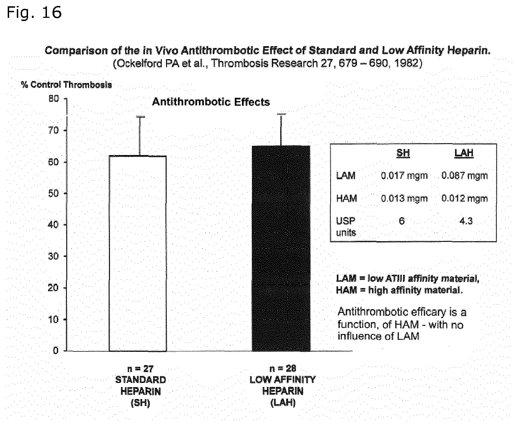

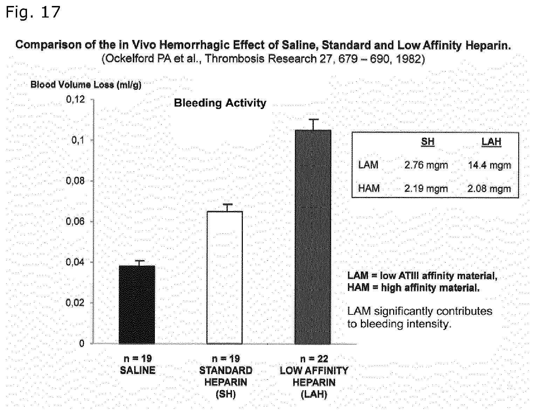

According to P.A. Ockelford "Low Affinity Material" as abundantly found in natural heparins - though anticoagulatorily in effective induce significant bleeding. That's proven by a series of sophisticated experiments of P.A.Ockelford who investigated upon the differential impact of HAM and LAM in terms of both their anti-thrombotic and bleeding-inducing potential. (Fig. 16, 17)

### Fig. 16

### Fig. 17

**The results demonstrate that antithrombotic effectiveness in indeed entirely dependent on HAM whereas half of the heparin-related excess blood loss proves to be due to LAM.**

What confers extra relevance to Ockelford's investigation is the fact that the ratio HAM/LAM of his experimental heparin species compares to that of enoxaparin.

***The basic rationale of LD Heparin's narrow molecular spectrum to a wide extent involves the exclusion of the low molecular weight faction and major amounts of LAM associated.***

As *another source of bleeding* of tantamount importance to the impact of LAM appears the fraction of *very long-chains in unfractionated heparin* (M>12 Kd) (Z 5, Fig. 2).

So it becomes explainable why UFH - *being substantially less burdened with* LAM than LMWH (50% vs 85%) - and the latter share a comparable bleeding risk just originating from two different sources.

**In LD Heparin both sources are drastically restrained thus combining superior efficacy and tolerance.**
**It is that *double-truncated molecular spectrum* that is to LD Heparin's specific advantage in the sensible indication of ischemic stroke.**

### Auxiliary properties of LD Heparin specifically supporting its indication in ischemic stroke

Co-morbidity of Ischemic Stroke and chronic renal disease. What it means for the positioning of LD Heparin

Recent studies have identified chronic renal disease (CRD) as a significant risk factor for atherosclerotic vasculo-pathies. While the association between CRD and cardio-vascular events is well established, reliable knowledge on the link with cerebrovascular lesion is of more recent date.

The association is really striking:
Due to a comprehensive Chinese study including >1000 patients with cerebrovascular lesions, mainly ischemic stroke, the total prevalence of CRD amounted to 48% ! (10)

The prevalence of stroke in the presence of CRD is a three times higher one.

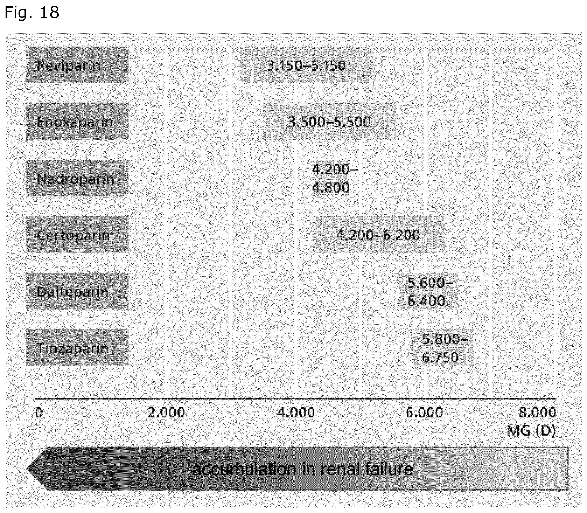

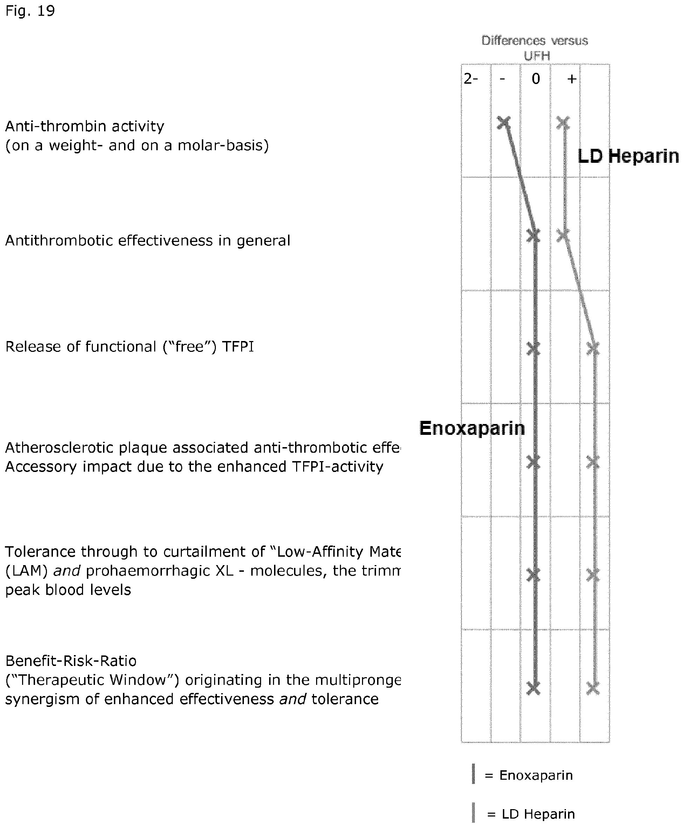

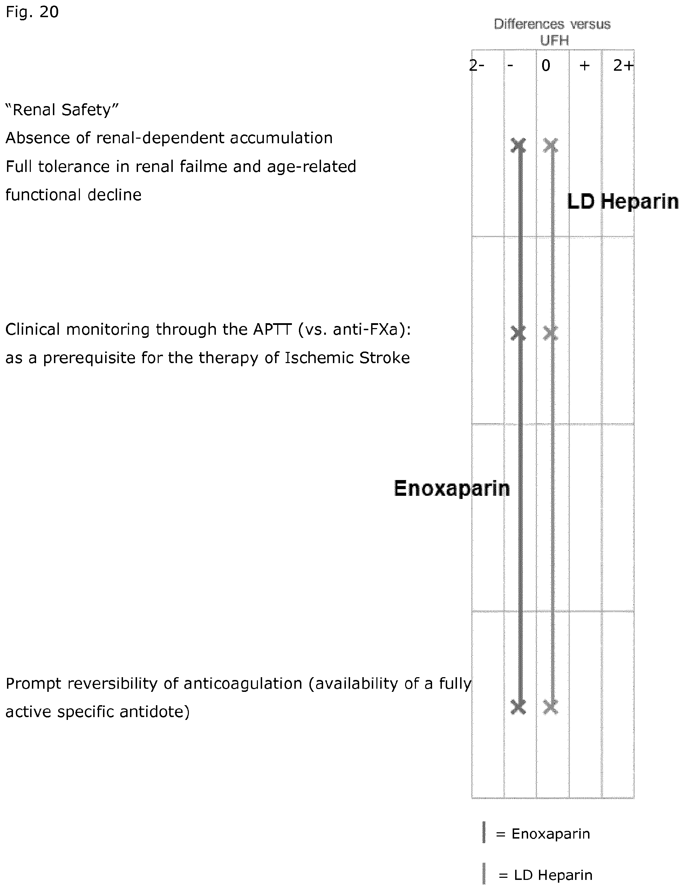

Multivariate logistic regression revealed that a reduced renal function as signalled by the "Glomerular Filtration Rate" (GFR) appears a significant predictor of mortality in acute stroke, and of poor outcome following discharge from hospital. The risk of stroke is 5 - 30% higher in patients with chronic kidney disease. (11) That risk of bleeding on heparins in advanced renal failme is more than doubled, which originates from plasmatic accumulation of the low molecular weight species of heparin, the renal-dependent accumulation being a proven molecular-weight-based process. (Fig.18)

### Fig. 18

The risk of bio-accumulation of LMWHs in renal failure - as a function of their molecular weight. (H. Schinzel, Vascular Care 12, 18-28,2007)

So it appears most favourable that LD Heparin doesn't accumulate in renal impairment of *any severity.* And no dose-correction would have to be considered not to mention the doubtful outcome of such measures after some increased mortality (thrombosis) had been found after lowering the heparin-doses applied. Reducing the dose of Enoxaparin by 50% at an age > 75 years and a GFR of 15-30 ml/min, this did not reduce the incidence of major bleeding. In contrast, lowering the dose to 0,57 U/ml, increased the 30-day mortality in the acute coronary syndrome. Controversially, elevated anti -Xa- levels did not predict major bleeding. (12)

### Renal safety exclusively fit in with the intended disease-specificity of the product.

Auxiliary properties continued .....

Monitoring of heparin as mandatory for treatment in Ischemic Stroke

Despite the limited evidence physicians have administered UFH to stroke patients for more than 50 years, and LMWH for at least 2 decades.

There is common consent, that the therapeutic hypotheses in stroke up to now have never been satisfactorily tested upon.

As the matter stands, such considerations lay significant stress upon *strict biological monitoring* of heparin in order to safeguard its narrow therapeutic margin.

That's the knowledge imparted through the prestigious monograph "Thrombolytic and Antithrombotic Therapy for Stroke," edited by J Bogousslavsky and W Hacke, Universities of Lausanne and Heidelberg. (13)

The monitorial instrumentation to assess upon the anticoagulatory effects of LD Heparin on the basis of the "APTT" ("Activated Partial Thrombin Time") - permits a *personalized* dose-adjustment and *dose-control.*

In contrast to that, and concretely in the framework of stroke-therapy, "excessive anticoagulation indicated by the APTT, frequently ended up in symptomatic and asymptomatic bleeding events". (13)

The so-called effect-kinetics of heparins as reflected by the given coagulation tests including the APTT are subject to *substantial variability* (Table 6) which once more spotlights the *necessity of a personalized APTT-control* in order to keep the individual anticoagulatory response within the applicable limits of 1,5 - 2,5 times the basis APTT-value.

In this way the treatment could be supervised on a "minute to minute" basis, including the possibility to intercept and reverse anticoagulation fully and instantaneous.

It's important to realize that for LMWH *no laboratory methods are available to assess upon the patient's response to anticoagulation,* the potential controls being restricted to the assessment of the *given heparin-concentration* in terms of the FXa antagonism.

It is the availability of a valid and sensitive monitoring instrument - now in our hands - which is factually permissive in using an anticoagulant agent for the therapy of stroke.

The usefulness of monitoring necessarily includes the possibility of countermeasures in case of some excessive anticoagulation or bleeding.

In this respect LD Heparin again takes a position of preference because its inhibitory effect can be easily reversed: It is that prompt and quantitative reversibility by protamin that makes up an important difference as against LMWH the anticoagulatory impact of which can be reversed by just 60%. (14)

LD Heparin 10,5 Kd at a Glance

The Comparative Positioning of LD Heparin and LMWH (Enoxaparin) as against UFH in the Framework of the Therapy for Ischemic Stroke.

### Fig. 19

### Fig. 20

References:
1.) Shakepousi MM, Monsavi's, et al. J Res Med Sci. 2012; 17 (4), 396. (Review)
2.) RP Xiao et al. N Engl J Med. 2017; 375.
3.) Sanderock PA, Counsel C, Kane E J. Anticoagulants for acute ischemik stroke . Conchrane Database Syst Rev. 2015; 3, CD000024.
4.) Fareed J, et al. J Thromb and Haemost. 1985; 11, 155-175.
5.) Cade JR, Buchanan MR Bonen B et al Thromb Research. 1989; 35, 613-625.
6.) Alban S, Welzel D and H C Hemker: Seminar's in Thrombosis and Hemostasis 28, 4; 369-377 (2002)
7.) Abildgaard U, Lindahl A K and Sandset P M. Haemostasis. 1991; 21, 254-57.
8.) Egan K, Connor H D et al. Thromb and Haemost. 2017; 8, 1549-58.
9.) Sandset PM. The Role of Tissue Factor Pathway Inhibitor ..... 15th Intern. Congress on Thrombosis, Antalya, 1998
10.) Xu S, Wang W, Shi H et al. Nephology Dialysis Transplantation. 2011; 26 (8), 2590-94.
11.) Nayak-Rao S, Shenoy M P Stroke in Patients with Chronic Kidney Disease ... (Review). Indian J of Nephology. 2017; 27 (3), 167-171.
12.) J Harenberg. Intern J Cardiol. 2016; 21251, 10-13.
13.) Thrombolytic and Antithrombotic Therapy for Stroke. Editors: J Bogousslavsky, W Hacke, Informa UK Ltd. 2006
14.) Hunt BJ. NEJM 2015; 370.

## Claims

1. Medium molecular weight heparin for use in the treatment and/or secondary prevention of ischemic stroke.

2. Medium molecular weight heparin according to claim 1, having an average molecular weight of more than 9 kD and less than 12 kD.

3. Medium molecular weight heparin according to claim 1, having an average molecular weight of more than 10 and less than 11 kD.

4. Medium molecular weight heparin according to claim 1, having an average molecular weight of 10.5 kD.

5. Use of medium molecular weight heparin as defined in anyone of claims 1 to 4 for the treatment and/or secondary prevention of ischemic stroke.
